# FASCICULE DE BREVET EUROPEEN

(11) **EP 0 973 808 B1**
(45) Date de publication et mention de la délivrance du brevet: **18.06.2003**
(21) Numéro de dépôt: 98921532.2
(22) Date de dépôt: 14.04.1998
(51) Int. Cl.: C08F 2/50, G03F 7/029

(54) **AMORCEURS NON TOXIQUES, RESINES A GROUPEMENTS ORGANOFONCTIONNELS RETICULABLES COMPRENANT LES AMORCEURS, ET LEUR UTILISATION POUR LA PREPARATION DE POLYMERES STABLES ET NON TOXIQUES**
NICHTTOXISCHER INITIATOR, HARZE MIT ORGANOFUNKTIONELLEN HÄRTBAREN GRUPPEN DIE DIESE INITIATOREN ENTHALTEN, UND DEREN VERWENDUNG IN DER HERSTELLUNG VON STABILEN, NICHT TOXISCHEN POLYMEREN
NON-TOXIC INITIATORS, RESINS WITH CROSS-LINKABLE ORGANOFUNCTIONAL GROUPS CONTAINING SAME, AND USE FOR PREPARING STABLE AND NON-TOXIC POLYMERS

(30) Priorité: 11.04.1997 FR 9704458
(43) Date de publication de la demande: 26.01.2000
(73) Titulaire: RHODIA CHIMIE, 92408 Courbevoie Cédex (FR)
(72) Inventeur: PRIOU, Christian, F-69100 Villeurbanne (FR); RICHARD, Jacques, F-38200 Luzinay (FR)
(86) Numéro de dépôt international: FR9800741
(87) Numéro de publication internationale: WO98046647

(56) Documents cités:
- DE-C- 4 110 793
- FR-A- 2 270 269

## Description

La présente invention a pour objet un nouveau procédé de préparation de résines non toxiques réticulables sous rayonnement en présence d'un système amorceur. Ces résines sont préparées à partir de compositions comprenant des monomères, oligomères et/ou polymères à groupements organofonctionnels, et sont réticulées en présence d'un système amorceur constitué par un sel d'onium de très faible toxicité.

Les nouvelles résines selon l'invention sont utilisées pour la réalisation d'encres non toxiques, de revêtements non toxiques anti-adhérants ou anti-adhésifs et pour la réalisation d'articles constitués d'un support solide dont une surface au moins est rendue anti-adhérante ou anti-adhésive par revêtement à l'aide desdites résines réticulables par rayonnement, notamment par activation photochimique ou par activation sous faisceau d'électrons.

Les sels d'onium ou de complexes organométalliques sont bien connus comme photoamorceurs de polymérisation cationique de monomères ou de polymères à groupements fonctionnels de type époxy, vinyléther. De nombreux documents décrivent ces photoamorceurs et leur utilisation: US-A-4.069.054; US-A-4.450.360; US-A-4.576.999; US-A-4.640.967; CA 1.274.646 ; EP-A- 203 829.

Toutefois, la plupart des sels amorceurs de l'art antérieur sont difficiles à manipuler et présentent en outre des risques de toxicité, ce qui limite donc leur domaine d'application et notamment l'utilisation des résines réticulées obtenues en présence de ces amorceurs dans des applications liées à l'industrie alimentaire, telles que l'emballage et le revêtement de plastiques et de métaux notamment, pour les conditionnements alimentaires.

La demanderesse a préparé un nouveau procédé de préparation de résines exempt de risque de toxicité qui met en oeuvre de nouveaux amorceurs judicieusement choisis et ne présentant pas les inconvénients de l'art antérieur, notamment en ce qui concerne la toxicité, ce qui permet ainsi l'utilisation desdits amorceurs dans des applications nécessitant une toxicité quasi nulle. Ainsi, de nouvelles résines ont été mises au point pour permettre leur utilisation dans des applications liées à l'industrie alimentaire, telles que l'emballage, et le revêtement de plastiques et de métaux. Ces nouvelles résines sont préparées à partir de compositions comprenant des monomères, oligomères et/ou polymères à groupements organofonctionnels, réticulables par apport d'énergie dont au moins une partie, de préférence la totalité, est fournie par un rayonnement U.V. de longueur d'onde comprise entre 200 et 400 nanomètres, en présence d'un système amorceur ; ce système amorceur est un sel d'onium non toxique dont la structure cationique est de formule:

[(CH(CH₃)₂-Φ-)-I-(-R¹)]⁺ (I)

dans laquelle le symbole R¹ représente le radical -Φ- R² , R² étant un radical alkyle linéaire ou ramifié comprenant de 1 à 20 atomes de carbone, de préférence 1 à 15 atomes de carbone.

De préférence, la structure cationique du sel d'onium est de formule [(CH(CH₃)₂-Φ-)-I-Φ-CH₃]⁺. En effet, les meilleurs résultats ont été obtenus avec cette structure; cette structure alliant à la fois une très faible toxicité et une activité optimale lors la réticulation des compositions réticulables.

La structure anionique du sel d'onium est choisie parmi le groupe constitué de Cl⁻, Br⁻, BF₄⁻, PF₆⁻, CF₃SO₃⁻, N(SO₂CF₃)₂⁻, C(SO2CF₃)₂⁻, B(C₆F₅)₄⁻ ,B(PhOCF₃)₄⁻ ,SbF₆⁻et/ou AsF₆⁻. Toutefois, les amorceurs suivants se sont avérés particulièrement adaptés dans la préparation de revêtements et/ou vemis non toxiques :

[CH₃-Φ-I-Φ- CH(CH₃)₂]⁺ Cl⁻, (CH₃-Φ-I-Φ- CH(CH₃)₂]⁺,B(C₆F₅)₄⁻,

[CH₃-Φ-I-Φ- CH(CH₃)₂]⁺ PF₆⁻, et [CH₃-Φ-I-Φ- CH(CH₃)₂]⁺B(PhOCF₃)₄⁻,

La réticulation peut être effectuée en présence de différents types de rayonnement, y compris thermique. En général, dans le cadre de la présente invention, cette réticulation est effectuée sous rayonnement Ultra-Violet (U.V.) et/ou sous rayonnement par faisceau d'électrons (E.B.).

Avantageusement, les amorceurs sont employés en solution dans un solvant organique (accélérateur), de préférence choisi parmi les donneurs de protons et, plus, préférentiellement encore, parmi les groupes suivants: alcools isopropyliques, alcools benzyliques, diacétone alcool, lactate de butyle et leur mélange.

Les amorceurs mis au point et utilisés dans le cadre de la présente invention peuvent être préparés par réaction d'échange entre un sel de l'entité cationique, par exemple un halogénure (chlorure, iodure), un hexafluorophosphate, un tétrafluoroborate, ou un tosylate avec un sel de métal alcalin (sodium, lithium, potassium) de l'entité anionique.

Les conditions opératoires, notamment les quantités respectives de réactifs, choix des solvants, durée, température, agitation, sont choisies pour permettre de récupérer l'amorceur selon l'invention sous forme solide par filtration du précipité formé ou sous forme huileuse par extraction à l'aide d'un solvant approprié.

Les sels de métal alcalin de l'entité anionique peuvent être préparés de manière connue, par réaction d'échange entre un composé halogénoboré et un composé organométallique (par exemple: magnésien, lithien, stannique) portant les groupements hydrocarbonés désirés, en quantité stoechiométrique, suivie éventuellement d'une hydrolyse à l'aide d'une solution aqueuse d'halogénure de métal alcalin; ce type de synthèse est par exemple décrite dans "J. of Organometallic Chemistry" vol 178, p.1-4, 1979 ; "J.A.C.S" 82, 1960, 5298 ; "Anal. Chem. Acta" 44, 1969, 175-183; US-A-4.139.681 et DE-A-2.901.367 ; "Zh. Org. Khim." Vol.25, N°5 - pages 1099-1102 Mai 1989.

Les tests mis en oeuvre pour la mesure de toxicité des amorceurs sont le test d'AMES et le test de sensibilisation L.L.N.A. Le test d'AMES permet de mesurer le pouvoir mutagène de l'amorceur et le test de sensibilisation LLNA permet de détecter le pouvoir sensibilisant de l'amorceur. Au cours de ce dernier test, la cytotoxicité des amorceurs peut être aussi détectée (voir exemples).

Les monomères et polymères utilisables dans le cadre de l'invention pour la préparation des résines à partir des compositions réticulables en accord avec l'invention sont de natures variées. Dans le cadre de l'invention, les essais ont été effectués avec des momomères et polymères de nature organique ou de nature silicone.

Parmi les monomères et polymères de nature silicone, les polymères définis ci-dessous sont utilisés dans le cadre de l'invention. Ces polymères sont des polyorganosiloxanes présentant des groupements fonctionnels de type époxy et/ou vinyléther.

Lesdits polyorganosiloxanes sont linéaires ou sensiblement linéaires et constitués de motifs de formule (II) et terminés par des motifs de formule (III) ou cycliques constitués de motifs de formule (II) représentées ci-dessous : 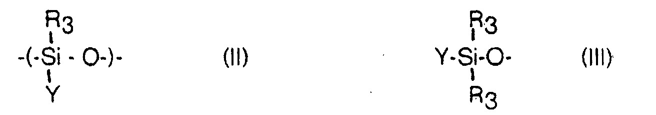 dans lesquelles :
- les symboles R₃ sont semblables ou différents et représentent :
   · un radical alkyle linéaire ou ramifié contenant 1 à 8 atomes de carbone, éventuellement substitué par au moins un halogène, de préférence le fluor, les radicaux alkyle étant de préférence méthyle, éthyle, propyle, octyle et 3,3,3-trifluoropropyle,
   · un radical cycloalkyle contenant entre 5 et 8 atomes de carbone cycliques, éventuellement substitué,
   · un radical aryle contenant entre 6 et 12 atomes de carbone pouvant être substitué, de préférence phényle ou dichlorophényle,
   · une partie aralkyle ayant une partie alkyle contenant entre 5 et 14 atomes de carbone et une partie aryle contenant entre 6 et 12 atomes de carbone, substituée éventuellement sur la partie aryle par des halogènes, des alkyles et/ou des alkoxyles contenant 1 à 3 atomes de carbone,
- les symboles Y sont semblables ou différents et représentent:
   · le groupement R₃, un radical hydrogène et/ou
   · un groupement organofonctionnel réticutable par voie cationique, de préférence un groupement époxyfonctionnel et/ou vinyloxyfonctionnel, relié au silicium du potyorganosiloxane par l'intermédiaire d'un radical divalent contenant de 2 à 20 atomes de carbone et pouvant contenir au moins un hétéroatome, de préférence de l'oxygène,
   · et l'un au moins des symboles Y représentant un groupement organique fonctionnel réticulable par voie cationique.

De préférence, au moins un des symboles R₃ des polyorganosyloxanes utilisés dans les compositions selon l'invention représente un radical phényle, tolyle ou dichlorophényle.

De plus, il est avantageux qu'au moins 60% molaire des radicaux R₃ des polyorganosyloxanes utilisés dans les compositions selon l'invention soient des radicaux méthyles.

Selon une variante préférée de l'invention, 1 à 50%, de préférence 5 à 25% des atomes de silicium du polyorganosiloxane porte 1 groupement fonctionnel réticulable.

A titre d'exemple, les groupements Y époxyfonctionnels sont généralement choisis parmi : 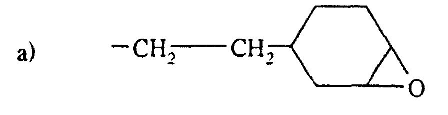 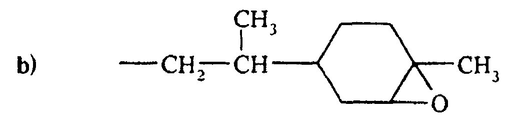 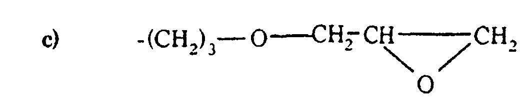 et/ou

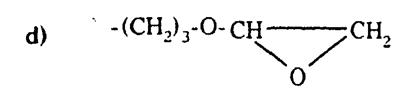

De préférence, les groupements fonctionnels Y correspondent à a).

En outre, les groupements Y vinyloxyfonctionnels sont généralement choisis parmi :
- (CH₂)₃-O-CH=CH₂
- -O-(CH₂)₄-O-CH=CH₂
- et/ou (CH₂)₃-O-R₄-O-CH=CH₂.
dans laquelle R₄ est :
- un alkylène linéaire ou ramifié en C₁-C₁₂ pouvant être substitué,
- un arylène en C₅-C₁₂, de préférence phénylène, pouvant être substitué, de préférence par 1 à 3 groupements alkyles en C₁-C₆.

Les polyorganosiloxanes époxy ou vinyloxyfonctionnels sont décrits notamment dans les références suivantes : DE-A- 4.009.889 ; EP-A- 396.130 ; EP-A- 355.381 ; EP-A- 105.341 ; FR-A- 2.110.115 ; FR-A- 2.526.800.

Les polyorganosiloxanes époxyfonctionnels peuvent être préparés par réaction d'hydrosilylation entre des huiles à motifs Si-H et des composés époxyfonctionnels tels que 1,2-époxy- 4 -vinyl-4 cyclohexane, allylglycidyléther.

Les polyorganosiloxanes vinyloxyfonctionnels peuvent être préparés par réaction d'hydrosilylation entre des huiles à motifs Si-H et des composés vinyloxyfonctionnels tels que l'allylvinyléther, l'allyl-vinyl oxyéthoxybenzène.

Les polyorganosiloxanes époxy ou vinyloxyfonctionnels se présentent généralement sous forme de fluides présentant une viscosité à 25°C de 10 à 10.000 mm²/s et de préférence de 100 à 600 mm²/s (viscosité mesurée avec viscosimètre BROOKFIELD, selon norme AFNOR NFT 76 102 de février 1972).

Parmi les monomères, oligomères et polymères de nature organique à groupements organofonctionnels, les polymères et monomères définis ci-dessous sont utilisés dans le cadre de l'invention. Ces monomères, oligomères, polymères comportent des fonctions réactives époxy, acrylate, alcényl-éther et/ou alcools.

Ces monomères, oligomères et/ou polymères de nature organique appartiennent à au moins l'une des espèces suivantes :
- α_{1.1}: époxydes cycloaliphatiques, pris à eux seuls ou en mélange entre eux :
- les époxydes du type 3,4-époxycyclohexylméthyl-3',4'-époxycyclohexane carboxylate: 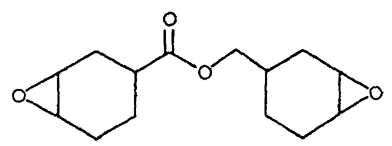
- ou Bis(3,4époxycyclohexyl)adipate, étant particulièrement préférés ;
- α_{1.2}: époxydes non cycloaliphatiques, pris à eux seuls ou en mélange entre eux :
- les époxydes du type de ceux résultant de la condensation de Bisphénol A et d'épichlorhydrine et du type :
   - di et triglycidyléthers de Bisphénol A alcoxylé de 1,6 hexane diol, de glycérol, de néopentylglycol et de propane triméthylol,
   - ou diglycidyléthers de Bisphénol A,
- les époxydes d'alpha-oléfines, époxydes NOVOLAC, huile de soja et de lin époxydés, polybutadiène époxydé, étant particulièrement préférés ;
- α₂: acrylates, pris à eux seuls ou en mélange entre eux ; e.g. :
- acrylates époxydés, de préférence l'oligomère de Bisphénol-A-époxydiacrylate (EBECRYL 600),
- acrylo-glycéro-polyester, de préférence mélange d'oligomère d'acrylate trifonctionnel obtenu à partir de glycérol et de polyester (EBECRYL 810),
- acrylates multifonctionnels, de préférence pentaérythritol triacrylate (PETA), triméthylol propane triacrylate (TMPTA), 1,6-hexanediol diacrylate (HDODA), triméthylolpropane éthoxylate triacrylate, thiodiéthylèneglycol diacrylate, tétra éthylène glycol diacrylate (TTEGDA), tripropylène-glycol diacrylate (TRPGDA), triéthylèneglycol diacrylate (TREGDA), triméthylpropane triméthacrylate (TMPTMA),
- acrylo-uréthanes,
- acrylo-polyéthers,
- acrylo-polyesters,
- polyesters insaturés,
- acrylo-acryliques,
étant particulièrement préférés ;
- α₃: alcényl-éthers, linéaires ou cycliques, pris à eux seuls ou en mélange entre eux :
- les vinyls-éthers, en particulier l'éther de triéthylène glycol divinylique (RAPIDCURE® CHVE-3, GAF Chemicals Corp.), les éthers vinyliques cycliques ou les tétramères et/ou dimères d'acroléines, et le vinyl-ether de formule suivante: 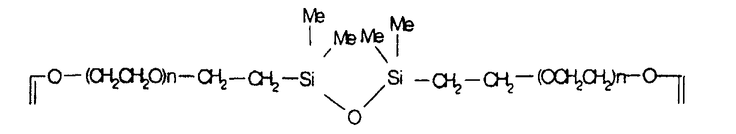
- les propényls-éthers,
- et les butényls-éthers étant, plus spécialement préférés,
- α₄: les polyols : pris à eux seuls ou en mélange entre eux, et de préférence le composé de formule ci dessous:

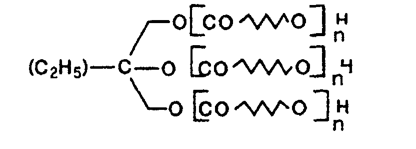

De plus, selon les applications envisagées (par exemple, vernis), les résines réticulables peuvent contenir non seulement des monomères, oligomères et/ou polymères de nature organique tels que définis ci-dessus mais contenir aussi, des monomères, oligomères et/ou polymères de nature silicone en accord avec la définition donnée précédemment.

Les compositions en accord avec l'invention peuvent comporter en outre d'autres ingrédients tels que des pigments, des modulateurs d'adhérence, des photosensibilisateurs des agents fongicides, bactéricides et anti-microbiens, des inhibiteurs de corrosion, etc. Ces compositions sont utilisables telles quelles ou en solution dans un solvant organique.

Les résines de l'invention préparées à partir des compositions comprenant des monomères, oligomères, et/ou polymères sont utilisables dans de nombreux domaines. En particulier, des encres non toxiques et des revêtements anti-adhérants et non toxiques sont fabriqués à base des résines selon l'invention et sont utilisés dans le domaine alimentaire du fait de leur faible toxicité (par exemple, contact entre des aliments et les encres et /ou revêtements).

En ce qui concerne les résines obtenues à partir des compositions comprenant des polyorganosiloxanes, ces dernières sont utilisables dans le domaine des revêtements anti-adhérents sur les matériaux cellulosiques, les peintures, l'encapsulation de composants électriques et électroniques, les revêtements pour textiles, ainsi que pour le gainage de fibres optiques. Elles sont tout particulièrement intéressantes lorsqu'elles sont utilisées telles quelles pour rendre un matériau non adhérent, tel que des feuilles métalliques, du verre, des matières plastiques ou du papier, à d'autres matériaux auxquels il adhérerait normalement. Dans ces domaines d'application, les résines à base de polyorganosiloxanes présentent avantageusement une viscosité ne dépassant pas 5 000 mPa.s, de préférence ne dépassant pas 4 000 mPa.s à 25°C.

Ainsi, les compositions réticulables à base de polyorganosiloxanes rendent des articles (feuilles par exemple) non adhérents à des surfaces auxquelles ils adhèrent normalement. Cette utilisation s'effectue par (a) application d'une quantité de la composition de l'invention, comprise généralement entre 0,1 et 5 g par m² de surface à enduire et (b) par réticulation de la composition par apport d'énergie dont au moins une partie, de préférence la totalité, est foumie par un rayonnement U.V.

Le rayonnement U.V. utilisé présente une longueur d'onde comprise entre 200 et 400 nanomètres, de préférence compris entre 254 et 360 nanomètres. La durée d'irradiation peut être courte, c'est à dire inférieure à 1 seconde et, de l'ordre de quelques centièmes de seconde pour les faibles épaisseurs de revêtements. La réticulation s'effectue en l'absence de chauffage ou en présence d'un chauffage entre 25 et 100°C. Le temps de durcissement se règle (a) par le nombre de lampes U.V. utilisées, (b) par la durée d'exposition aux U.V. et/ou (c) par la distance entre la composition et la lampe U.V.

Les compositions à base de polyorganosiloxanes sans solvant, c'est-à-dire non diluées, sont appliquées à l'aide de dispositifs aptes à déposer, d'une façon uniforme, de faibles quantités de liquides. On peut utiliser à cet effet par exemple le dispositif nommé "Helio glissant" comportant en particulier deux cylindres superposés : le rôle du cylindre placé le plus bas, plongeant dans le bac d'enduction où se trouve la composition, est d'imprégner en une couche très mince le cylindre placé le plus haut, le rôle de ce dernier est alors de déposer sur le papier les quantités désirées de composition dont il est imprégné, un tel dosage est obtenu par réglage de la vitesse respective des deux cylindres qui tournent en sens inverse l'un de l'autre.

Les quantités de compositions déposées sur les supports sont variables et s'échelonnent le plus souvent entre 0,1 et 5 g/m² de surface traitée. Ces quantités dépendent de la nature des supports et des propriétés anti-adhérentes recherchées. Elles sont plus souvent comprises entre 0,5 et 1,5 g/m² pour des supports non poreux.

La présente invention a également pour objet les articles (feuilles par exemple) constituées d'un matériau solide (métal, verre, matière plastique, papier, etc.) dont une surface au moins est revêtue de résine de nature organique et/ou de nature silicone, telle que définie précédemment, réticulée par rayonnement ultra violet ou réticulée par rayonnement sous faisceau d'électrons.

### Exemples et tests

Les exemples et tests suivants sont donnés à titre illustratif et ne peuvent être considérés comme une limite du domaine et de l'esprit de l'invention.

### I. Préparations de sel d'iodonium selon l'invention et selon l'art antérieur.

### Exemple 1 : synthèse du sel d'iodonium

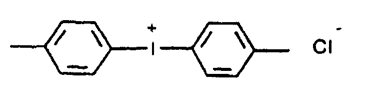

Dans un ballon tétracol de 500 ml, muni d'un agitateur mécanique, d'une ampoule de coulée, d'un réfrigérant à eau, et d'un thermomètre, sont placés la glace (6,35 g) et l'acide sulfurique (20 g, 0,2 mole). Le mélange est refroidi à 20°C, puis l'acide acétique glacial (30 g, 0,5 mole) est ajouté. Le persulfate d'ammonium (41 g, 0,18 mole) est ensuite chargé à 20°C ± 5°C. Le milieu réactionnel est alors refroidi à 15°C et agité à une vitesse de 420 tr/mn. Enfin, le mélange de 4-iodotoluène (21,8 g; 0,1 mole) dans le toluène (36,8 g; 0,4 mole) est additionné en 2 heures, en maintenant la température à 20°C.

Après addition de tous les réactifs, l'agitation est maintenue à 20°C pendant 16 à 20 heures, la fin de la réaction étant déterminée par CCM (éluant: CCl₄). La précipitation du chlorure de ditolyliodonium se fait par addition d'une solution de NaCl (35 g dans 100 ml d'eau). Le mélange est filtré et le précipité jaune est lavé avec une solution saturée de NaCl puis avec de l'eau afin d'éliminer l'acide acétique restant. Le solide est ensuite lavé avec du toluène puis du pentane.

Après séchage dans une étuve sous vide. Le chlorure de ditolyliodonium est récupéré. Il est ensuite purifié par recristallisation dans l'acétone à chaud. Le rendement est de 46 %.

Analyses : Tf = 188°C ; RMN ¹H (300mhz) : δ=2,24 (s,6H, Me); 7,92 (d, 4H, ArH).

### Exemple 2 : synthèse du sel d'iodonium :

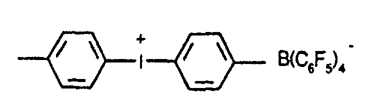

Dans un erlenmeyer de 250 ml équipé d'une agitation magnétique, on charge le tétrakis(pentafluorophényl)borate de potassium (14,4 g, 0,02 mole), le chlorure de ditolyliodonium (6,9 g, 0,02 mole) et le méthanol (90 g). Ce mélange est agité pendant 1 h à température ambiante, puis filtré sur fritté n°4 pour éliminer le chlorure de potassium formé pendant la réaction. La solution est reprise dans un erlenmeyer équipé d'une agitation magnétique, puis l'eau (42 g) est coulée goutte à goutte en 20 mn. Le mélange est ensuite maintenu pendant 30 mn sous agitation. On filtre sur fritté n°4, puis le produit obtenu est séché pendant 24 h à 50°C sous une pression de 18 mm Hg. On récupère 16,8 g d'une poudre blanche, soit un rendement de 85%.

Analyses : HPLC : Anion 69,6%, cation 28,7 % ; teneur en eau : 0,035 %; teneur en chlorures : 0,04%.

### Exemple 3 : synthèse du sel d'iodonium :

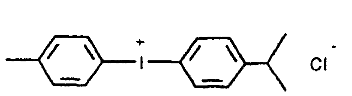

Dans un ballon tétracol de 500 ml, muni d'un agitateur mécanique, d'une ampoule de coulée, d'un réfrigérant à eau, et d'un thermomètre, sont placés la glace (6,33 g) et l'acide sulfurique (20,8 g, 0,2 mole). Le mélange est refroidi à 20°C, puis l'acide acétique glacial (30 g, 0,5 mole) est ajouté. Le persulfate d'ammonium (41 g, 0,18 mole) est ensuite chargé à 20°C ± 5°C. Le milieu réactionnel est alors refroidi à 15°C et agité à une vitesse de 420 tr/mn. Enfin, le mélange de 4-iodotoluène (21,8 g; 0,1 mole) dans le cumène (24,42 g; 0,2 mole) est additionné en 2 heures, en maintenant la température à 20°C.

Après addition de tous les réactifs, l'agitation est maintenue à 20°C pendant 16 à 20 heures, la fin de la réaction étant déterminée par CCM (éluant: CCl₄). La précipitation du chlorure de cumyltolyliodonium se fait par addition d'une solution de NaCl (35g dans 100 ml d'eau). Le mélange est filtré et le précipité jaune est lavé avec une solution saturée de NaCl puis avec de l'eau afin d'éliminer l'acide acétique restant. Le solide est ensuite lavé avec du toluène puis du pentane. Après séchage dans une étuve sous vide. Le chlorure de cumyltolyliodonium est récupéré. Il est ensuite purifié par recristallisation dans l'acétone à chaud. le rendement est de 75 %.

Analyses : Tf = 188,6°C ; RMN ¹H (300mhz) : δ=1,09 (d,6H, Me); 2,24 (s, 3H, Me); δ=2,82 (m,1H, CH); 7,17 (d, 2H, ArH); δ=7,24 (d,2H, ArH); 7,94 (dd, 4H, ArH).

### Exemple 4 : synthèse du sel d'iodonium :

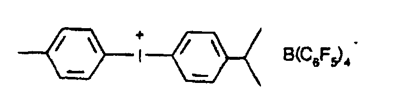

Dans un erlen meyer de 100 ml équipé d'une agitation magnétique, on charge le tétrakis(pentafluorophényl)borate de potassium (18 g, 0,025 mole), le chlorure de cumyltolyliodonium (9,31 g, 0,025 mole) et le méthanol (110 g). Ce mélange est agité pendant 2 h à température ambiante, puis filtré sur fritté n°4 pour éliminer le chlorure de potassium formé pendant la réaction. La solution est reprise dans un erlen meyer équipé d'une agitation magnétique, puis l'eau (150 ml) est coulée goutte à goutte en 20 mn. Le mélange est ensuite maintenu pendant 30 mn sous agitation. On filtre sur fritté n°4, puis le produit obtenu est lavé par 3 fractions d'eau (3x100 ml), puis est séché pendant 24 h à 50°C sous une pression de 20 mm Hg. On récupère 15,6 g d'une poudre blanche, soit un rendement de 61%.

Analyses : HPLC : Anion 56,9 %, cation 40,5 %.

### Exemple 5 : synthèse de l'anion :

KB(PhOCF₃)₄

Dans un réacteur de 600 ml à double enveloppe, équipé d'une agitation mécanique, d'un réfrigérant contenant de la carboglace et de l'acétone, et d'une ampoule de coulée, on charge l'éther isopropylique (200 ml) et le bromo-4-trifluoroanysol (51,6 g; 214,2 mmole). Le montage est soigneusement inerté à l'argon puis, l'hexyl lithium à 33% dans l'hexane (56,8 g, 203 mmole) est chargé sous atmosphère inerte dans l'ampoule de coulée. Le mélange réactionnel est refroidi à la température de -70°C par circulation dans la double enveloppe d'un mélange carboglace-acétone. L'hexyl lithium est alors ajouté au goutte à goutte en 20 mn. La masse réactionnelle se colore en orange. L'ampoule de coulée est remplacée par une nouvelle ampoule de coulée contenant le trichlorure de bore en solution 1M dans l'heptane (34,1 g, 46,1 mmole). La coulée dure 15 mn, puis on vidange la double enveloppe du réacteur et on laisse la masse réactionnelle se réchauffer. A la température de 0°C, on coule sur la masse réactionnelle une solution aqueuse saturée en chlorure de potassium (200 ml). Le mélange est agité pendant 15 mn, puis l'agitation est arrêtée et les phases sont séparées. On ajoute à la phase organique 250 ml de solution aqueuse saturée en KCl puis on distille sous pression réduite (10 mm Hg ) les produits organiques jusqu'à la température d'ébullition de l'eau. Au cours de la distillation, le tétrakis(trifluoroanisyl)borate de potassium précipite. Il est recueilli après refroidissement de la masse réactionnelle par filtration. Le solide blanc obtenu est séché pendant 24h à la température de 50°C sous un vide de 20 mm Hg. On récupère 26,8 g de produit, soit un rendement de 84 %.

Analyses : Tf : > 300°C (dec) ; RMN ¹H (300 mhz) : pureté 95% (mol); RMN ¹⁹F (282 mhz) : δ=19,97.

### Exemple 6 : synthèse du sel d'iodonium

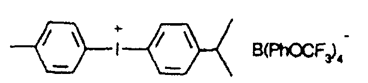

Dans un erlen meyer de 100 ml équipé d'une agitation magnétique, on charge le tétrakis(trifluoroanisyl)borate de potassium (7,14 g, 0,020 mole ), le chlorure de cumyltolyliodonium (3,83 g, 0,020 mole) et le méthanol (40 g). Ce mélange est agité pendant 2 h à température ambiante, puis est filtré sur fritté n°4 pour éliminer le chlorure de potassium formé pendant la réaction. La solution est reprise dans un erlen meyer équipé d'une agitation magnétique, puis est coulée sur un pied d'eau (300 ml). On ajoute ensuite une solution aqueuse saturée en chlorure de potassium (100 ml) pour faire flocuter le solide blanc. On filtre sur fritté n°4, puis le produit obtenu est lavé par 1 fraction d'eau (500 ml), puis est séché pendant 24 h à 50°C sous une pression de 20 m Hg. On récupère 8,4 g d'une poudre blanche, soit un rendement de 83 %.

Analyses : HPLC : Anion 64,2 %, cation 30,4 %.

### Exemple 7 : synthèse du sel d'iodonium:

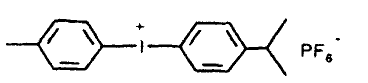

Dans un erlen meyer de 100 ml équipé d'une agitation magnétique, on charge l'hexafluorophosphate de sodium (6,7 g, 0,040 mole), le chlorure de cumyltolyliodonium (14,9 g, 0,040 mole) et le méthanol (80 g). Ce mélange est agité pendant 1 h à température ambiante, puis est filtré sur fritté n°4 pour éliminer le chlorure de sodium formé pendant la réaction. La solution est reprise dans un erlen meyer équipé d'une agitation magnétique, puis est coulée sur un pied d'eau (600 ml). On ajoute ensuite une solution aqueuse saturée en chlorure de potassium (50 ml) pour faire floculer le solide blanc. On filtre sur fritté n°4, puis le produit obtenu est lavé par 3 fractions d'eau (100 ml), puis est séché pendant 24 h à 45°C sous une pression de 20 mm Hg. On récupère 14,4 g d'une poudre légèrement brune, soit un rendement de 75 %.

### Exemple 8 : synthèse du sel d'iodonium :

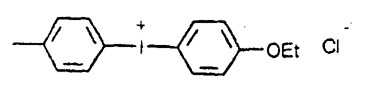

Dans un tétracol de 500 ml, muni d'un agitateur mécanique, d'une ampoule de coulée, d'un réfrigérant à eau, et d'un thermomètre, sont placés la glace (6,33 g) et l'acide sulfurique (21,1 g, 0,2 mole). Le mélange est refroidi à 20°C, puis l'acide acétique glacial (30 g, 0,5 mole) est ajouté. Le persulfate d'ammonium (42 g, 0,184 mole ) est ensuite chargé à 20°C ± 5°C. Le milieu réactionnel est alors refroidi à 15°C et agité à une vitesse de 420 tr/mn. Enfin, le mélange de 4-iodotoluène (21,9 g; 0,1 mole) dans l'éthoxybenzène (24,45 g; 0,2 mole) est additionné en 2 heures, en maintenant la température à 20°C.

Après addition de tous les réactifs, l'agitation est maintenue à 20°C pendant 16 à 20 heures, la fin de la réaction étant déterminée par CCM (éluant : CCl₄). La précipitation du chlorure de phénétylitolyliodonium se fait par addition d'une solution de NaCI (35g dans 100 cc d'eau). Le mélange est filtré et le précipité jaune est lavé avec une solution saturée de NaCI puis avec de l'eau afin d'éliminer l'acide acétique restant. Le solide est ensuite lavé avec du toluène puis du pentane. Après séchage dans une étuve sous vide, le chlorure de phénétyltolyliodonium est récupéré. Il est ensuite purifié par recristallisation dans l'acétone à chaud. Le rendement est de 56 %.

Analyses : Tf = 212,8°C ; RMN ¹H (300mhz) : δ=1,14 (t,3H, Me); 2,05 (s, 3H, Me); δ=3,77 (q,2H, CH2); 6,85 (d, 2H, ArH); δ=7,03 (d,2H, ArH); 8,04 (d, 2H, ArH); 8.07 (d. 2H. ArH).

### Exemple 9 : synthèse du sel d'iodonium :

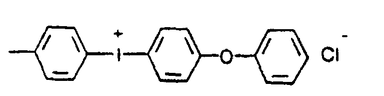

Dans un tétracol de 500 ml, la glace, l'acide sulfurique et le persulfate d'ammonium sont chargés de la même façon que dans l'exemple 2. Le mélange est refroidi à 15°C et agité à 420 tr/mn, puis la solution de 4-iodotoluène (22 g; 0,1 mole) et de diphényléther (17,2 g; 0,05 mole) dans l'acide acétique (32 ml) est additionnée en deux heures à la température de 20°C.. La réaction terminée, une solution de NaCl (35 g dans 100 g d'eau) est ajoutée. Le produit est récupéré par filtration, et se présente sous la forme d'une pâte rouge qui se solidifie au séchage. Ce solide est abondamment lavé par une solution saturée de chlorure de sodium, puis il est repris dans l'acétone à chaud. Après refroidissement de l'acétone, le produit est récupéré par filtration. On récupère ainsi 32,7 g d'un solide légèrement jaune, soit un rendement de 80%.

Analyses : Tf = 189,1°C ; RMN ¹H (300mhz): δ=2,25 (s,3H, Me); 6,91 (d, 2H, ArH); δ=7,01 (d,2H, ArH); 6,85 (d, 2H, ArH); δ=7,16 (t,1H, ArH); 7,20 (d, 2H, ArH); 7,37 (dd, 2H, ArH); 7,94 (d, 2H, ArH); 8,02 (d, 2H, ArH).

### Exemple 10 : synthèse du sel d'iodonium:

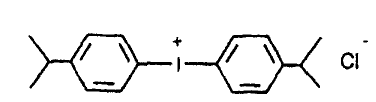

Le sel d'iodonium 10 est préparé selon le procédé décrit dans l'exemple 1 pour le chlorure de ditolyliodonium, en remplaçant le iodotoluène par du iodocumène, et le toluène par du cumène. On obtient le sel d'iodonium avec un rendement de 48%.

Analyses : Tf = 176.5°C ; RMN ¹H (300mhz): δ=1,14 (d,12H, Me); 2,88 (m,2H, CH); 7,32 (d, 4H, ArH); 8.05 (d, 4H, ArH).

### Exemple 11 : synthèse du sel d'iodonium :

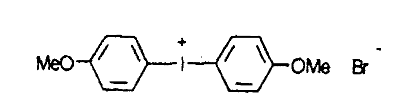

Le sel d'iodonium 11 est préparé selon le procédé décrit dans l'exemple 1 pour le chlorure de ditolyliodonium, en remplaçant le iodotoluène par du iodoanisol, et le toluène par de l'anisol. On obtient le produit attendu avec un rendement de 30%.

### Exemple 12: synthèse du sel d'iodonium :

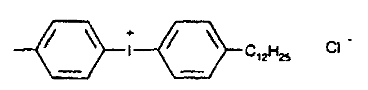

Le sel d'iodonium 12 est préparé selon le procédé décrit dans l'exemple 1 pour le chlorure de ditolyliodonium, en remplaçant le toluène par du dodécylbenzène. On obtient le produit attendu avec un rendement de 55%.

Analyses : Tf = 132°C ; RMN ¹H (300mhz) : δ=0.85 (t,3H,Me); 1,22 (m, CH₂); 1,44 (m, CH₂); 2,08 (s,3H,Me); 2,47 (t,2H,CH2); 7,04 (d, 2H, ArH);7,19 (d, 2H, ArH), 8,11 (d, 2H, ArH); 8,20 (d, 2H, ArH).

### II. Tests de toxicologie des amorceurs.

Les tests de toxicologie suivants ont été effectués:
- Test d'AMES: ce test d'AMES permet de mesurer le pouvoir mutagène du produit. • Test de sensibilisation LLNA : ce test permet de détecter le pouvoir sensibilisant du produit. Au cours de ce test, la cytotoxicité des produits peut être aussi détectée.

Le Tableau I ci-après indique les résultats obtenus pour les différents photoamorceurs préparés ci-dessus.

Les tests ont permis de déterminer les structures des photoamorceurs répondant négativement au test d'AMES et négativement au test sensibilisant LLNA.

Dans le cadre des tests, tous les photoamorceurs non toxiques se caractérisent par la présence de la structure non symétrique suivante :

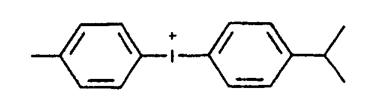

Les autres photoamorceurs testés sont positifs au test de sensibilisation LLNA, et/ou positif au test de AMES et/ou éventuellement cytotoxique.

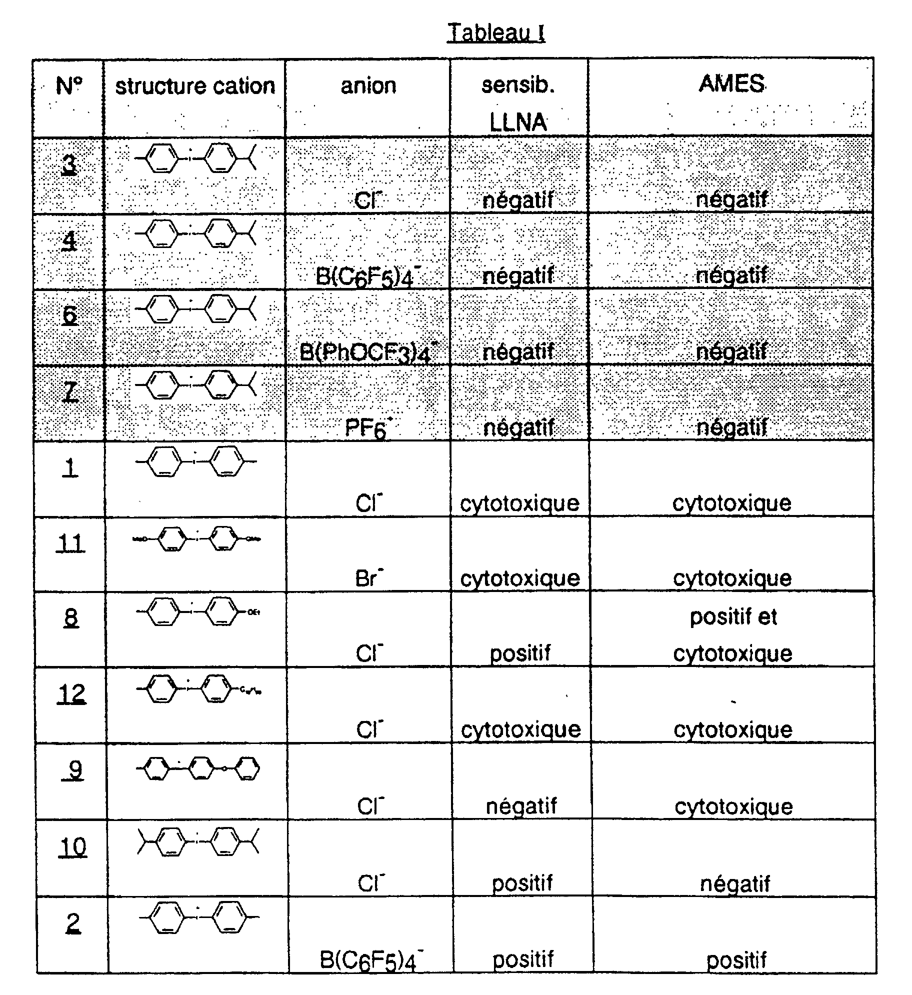

### III. Préparation de résines réticulables comprenant des polymères siliconés.

Des résines en accord avec l'invention, comprenant des polymères siliconés (matrice silicone époxy) ont été préparées.

Les compositions réticulables comprennent l'amorceur non toxique préparé à l'exemple 4. Le photoamorceur de l'exemple 4, comme ceux des exemples 3, 6 et 7, présentent une réactivité sous irradiation U.V. comparable aux autres photoamorceurs toxiques.

### A. Préparation de la composition réticulable.

On mélange:
- 100 parties en poids du polyorganosiloxane fonctionnalisé mis en oeuvre dans la présente préparation est le (1,2-époxy-4-éthyl-cyclohexyl)polydiméthylsiloxane de formule ci-dessous dans laquelle a et b ont respectivement pour valeurs moyennes 7 et 73. 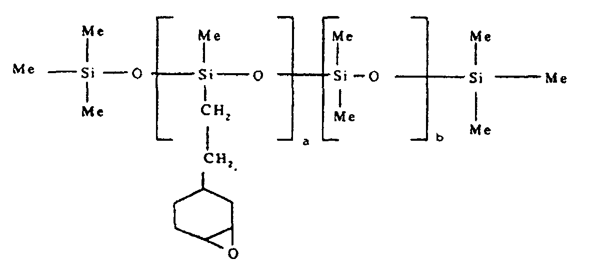
- et 0,5 partie en poids d'amorceur préparé à l'exemple 4 mis en solution à 20% en poids dans du 4-hydroxy-4-méthyl-2-pentanone.

### B. Evaluation des revêtements anti-adhérants à base de résines réticulées.

Après agitation pendant 30 minutes, la composition réticulable est déposée (environ 0,5 à 3 g/m³) sur un papier couché polyéthylène PEK 40/12 commercialisé par la société LONJAN.

Le papier enduit passe sous une lampe U.V. de technologie FUSION SYSTEM F450 caractérisée par:
- une longueur d'onde de 360 nm,
- une absence d'électrodes,
- une puissance de 120W/cm irradié.

La vitesse de défilement du papier sous la lampe est de 200 m/mn.

Après passage sous la lampe, la qualité du revêtement obtenue après durcissement est évaluée par mesure des propriétés anti-adhérentes de la couche silicone après avoir été mise en contact :
(i) avec un adhésif acrylique TESA 4970:
   - pendant 20 heures à 23°C,
   - pendant 20 heures à 70°C,
   - pendant 7 jours à 70°C.
(ii) avec un adhésif caoutchouc TESA 4154 pendant 20 heures et 15 jours à 23°C
(iii) avec un adhésif caoutchouc TESA 4651 pendant 20 heures et 15 jours à 23°C

Les résultats obtenus figurent au tableau II. Les revêtements obtenus présentent des forces d'adhérence extrêmement stable, même après vieillissement accéléré via augmentation de la température.

D'autre part, on mesure le taux de produit organosiliconé extractible dans le revêtement , après extraction pendant 24 heures dans du toluène. On retrouve seulement 2,2% de silicone dans la phase tolunéique, ce qui est révélateur d'une bonne réticulation de la résine.

**Tableau II**

| | TESA 4154 | TESA 4651 | TESA 4970 |
|---|---|---|---|
| 20 h / 23°C | 2.2 q / cm | 6.2 g / cm | 6.9 g / cm |
| 15 jours/ 23°C | 2.7 g / cm | 6.8 g / cm | 8.2 g / cm |
| 20 h / 70°C | / | / | 9.0 g / cm |
| 7 jours / 70°C | / | / | 13.7 g / cm |

### IV. Préparation de résines à base des compositions réticulables pour la fabrication d'encres blanches.

Les compositions réticulables testées comprennent soit le photoamorceur P1 ou le photoamorceur P2. Ces résines avec P1 ou P2 sont comparées avec les résines réticulées en présence du photoamorceur de la société SARTOMER P3 .

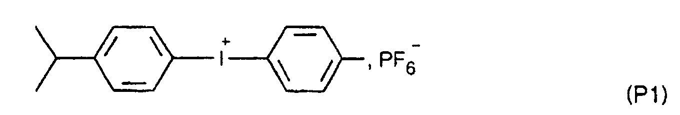

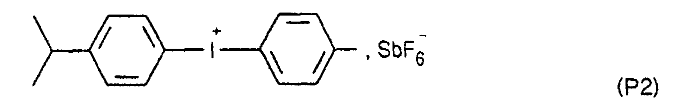

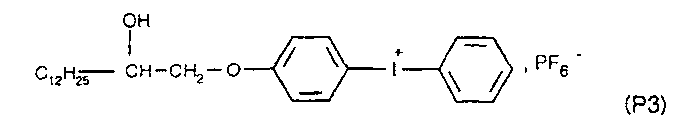

### A. Préparation des compositions réticutables.

1. Dans un réacteur de capacité 2 litres muni d'une agitation centrale tripale, une base pigmentaire concentrée ( B.P.C) est obtenue par dispersion de :
- 700 parties d'oxyde de titane de type rutile commercialisé par la société Dupont de Nemours,
- 3,5 parties de dispersant Solsperse 24000C commercialisé par la société Zeneca, et
- 296,5 parties de résine époxyde cycloaliphatique M1 commercialisée sous le nom CYRACURE 6110 ou 6105 par UnionCarbide:

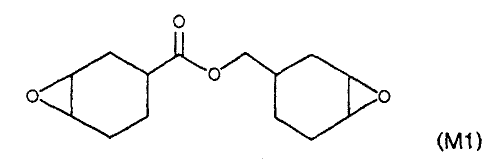

Cette base pigmentaire concentrée est obtenue par mélange pendant 30 minutes après coulée de la poudre d'oxyde de titane sur le mélange résine/dispersant préchauffé à 40°C. Puis, la base pigmentaire concentrée est formulée avec le photoamorceur et les additifs tels que des agents de nivellement (BYK361 par exemple) ou un agent flexibilisant tel que le produit T1 commercialisé par UNION-CARBIDE: 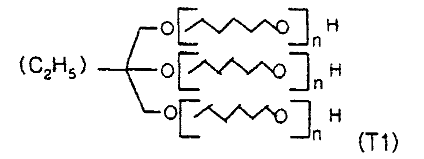
2. Les photoamorceurs avant d'être ajoutés à la base pigmentaire sont mis en solution en présence du photosensibilisateur appartenant à la famille des thioxanthones. Dans le cas présent, on utilise le chloro1-propoxy-4-thioxanthone (CPTX).
Les solutions sont réalisées par mélange à 20°C avec de l'isopropanol et du vinyléther DVE-3 commercialisé par la société ISP de formule: 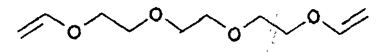
Ce vinyléther permet une très bonne dilution des composés et une très bonne stabilité des solutions conservées à l'abri de la lumière.
Le mélange s'effectue sous agitation magnétique pendant une heure à l'abri de la lumière. On obtient des solutions limpides que l'on conserve à l'abri de la lumière.
Le tableau III indique les parts de chaque constituant dans les solutions d'isopropanol et de vinyléther S1 à S9.

**Tableau III**

| Réactifs | S1 | S2 | S3 | S4 | S5 | S6 | S7 | S8 | S9 |
|---|---|---|---|---|---|---|---|---|---|
| DVE-3 (parts) | 12 | 12 | 12 | 12 | 12 | 12 | 12 | 12 | 12 |
| P1 | 4 | 0 | 0 | 4 | 0 | 0 | 4 | 0 | 0 |
| P2 | 0 | 4 | 0 | 0 | 4 | 0 | 0 | 4 | 0 |
| P3 | 0 | 0 | 4 | 0 | 0 | 4 | 0 | 0 | 4 |
| Isopropanol | 4 | 4 | 4 | 4 | 4 | 4 | 4 | 4 | 4 |
| CPTX | 1,2 | 1,2 | 1,2 | 1,6 | 1,6 | 1,6 | 1,92 | 1,92 | 1,92 |

3. Ces solutions S1 à S9 sont alors mélangées avec la base pigmentaire concentrée. Les pourcentages des constituants des compositions réticulables F1 à F9 sont donnés dans le tableau IV ci dessous.

**Tableau IV**

| Référence | F1 | F2 | F3 | F4 | F5 | F6 | F7 | F8 | F9 |
|---|---|---|---|---|---|---|---|---|---|
| B.P.C% | 81,7 | 81,7 | 81,7 | 81,7 | 81,7 | 81,7 | 81,7 | 81,7 | 81,7 |
| S1 % | 10,6 | 0 | 0 | 0 | 0 | 0 | 0 | 0 | 0 |
| S2% | 0 | 10,6 | 0 | 0 | 0 | 0 | 0 | 0 | 0 |
| S3% | 0 | 0 | 10,6 | 0 | 0 | 0 | 0 | 0 | 0 |
| S4% | 0 | 0 | 0 | 8,1 | 0 0 | 0 | 0 | 0 | 0 |
| S5% | 0 | 0 | 0 | 0 | 8,1 | 0 | 0 | 0 | 0 |
| S6% | 0 | 0 0 | 0 | 0 | 0 | 8,1 | 0 | 0 | 0 |
| S7% | 0 | 0 | 0 | 0 | 0 | 0 0 | 6,85 | 0 | 0 |
| S8% | 0 | 0 | 0 | 0 | 0 | 0 | 0 0 | 6,85 | 0 |
| S9% | 0 | 0 | 0 | 0 | 0 | 0 | 0 | 0 | 6,85 |
| M1% | 2,2 | 2,2 | 2,2 | 4,7 | 4,7 | 4,7 | 5,95 | 5,95 | 5,95 |
| T1 % | 4 | 4 | 4 | 4 | 4 | 4 | 4 | 4 | 4 |
| BYK361 % | 1,5 | 1,5 | 1,5 | 1,5 | 1,5 | 1,5 | 1,5 | 1,5 | 1,5 |

### B. Evaluation des encres obtenues à base des résines réticulées de F1 à F9.

Le séchage des encres est évalué sur un banc U.V. de la société IST Gmbh muni d'une lampe au mercure dopée au gallium et d'une lampe au mercure. Les lampes ont une puissance de 180W/cm et ont été étalonnées à l'aide d'une cellule Powerpuck de la société E.I.T. de façon à déterminer l'intensité et la dose reçue en fonction de la vitesse de défilement du banc.
- A la vitesse de défilement de 10 m/mn, chaque formulation reçoit :
   - UVA (320-390nm) =0,641 J/cm² ; UVB (280-320nm)=0,564 J/cm²;
   - UVC (250-260nm) =0,078J/cm² ; et UVV (390-440nm)=0,1255J/cm²
- A la vitesse de défilement de 20 m/mn, chaque formulation reçoit:
   - UVA (320-390nm) =0,314J/cm² ; UVB (280-320nm)=0,276 J/cm²
   - UVC (250-260nm) =0,033J/cm² ; et UVV (390-440nm)=0,781 J/cm²
- A la vitesse de défilement de 50 m/mn, chaque formulation reçoit :
   - UVA (320-390nm) =0,100J/cm² ; UVB (280-320nm)=0,092J/cm²
   - UVC (250-260nm) =0,012J/cm² ; et UVV (390-440nm)=0,599J/cm².

(i) On mesure la résistance aux solvants des encres obtenues après séchage de films de 12 µm±3 sur support aluminium par le nombre d'aller-retours, effectués à l'aide d'un chiffon imbibé de solvant, ce nombre est celui nécessaire pour désagréger la couche d'encre après 1 heure et après 24 heures . Dans le cas présent, le solvant utilisé est la méthyléthylcétone (MEK).
(ii) On mesure également la flexibilité des couches selon la méthode de T-Bend . La plaque d'une épaisseur de 250 µm est repliée sur elle- même . Si on ne note pas d'enlèvement de la couche d'encre après arrachement au scotch sur la tranche, on note T0. Si le revêtement se craquelle ou se déchire et /ou est arraché par le scotch, on replie une deuxième fois la plaque et on effectue les mêmes observations. Si le revêtement n'est pas affecté par ce second pliage, on note que le produit répond à T0,5, valeur correspondant au rapport du rayon de courbure à l'épaisseur. Si le test est négatif, on procède ainsi jusqu'à T3 soit un rayon de courbure égal à trois fois l'épaisseur. Au-delà on considère que la couche d'encre est fragile et peu flexible.
(iii) On mesure également l'adhérence notée de 0 à 5 selon la méthode normalisée de "*crosshatch adhésion*". 0 = 100% d'adhésion.

Les résultats des évaluations sont donnés dans le Tableau V ci après.

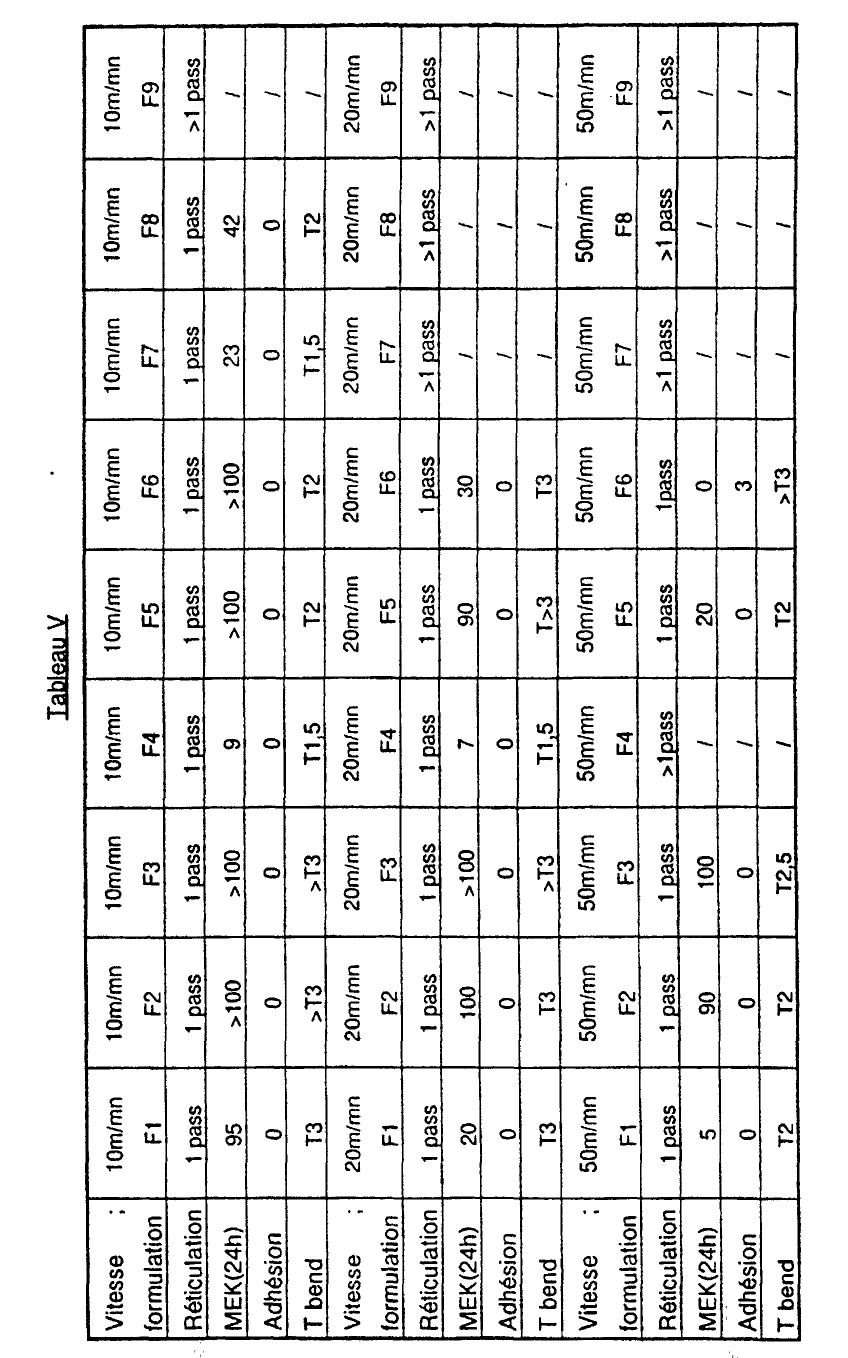

## Revendications

1. Procédé de préparation de résines non toxiques à partir de compositions comprenant des monomères, oligomères et/ou polymères à groupements organofonctionnels, réticulables par apport d'énergie dont au moins une partie, de préférence la totalité, est fournie par un rayonnement U.V. de longueur d'onde comprise entre 200 et 400 nanomètres, en présence d'un système amorceur, **caractérisé en ce que** le système amorceur est un sel d'onium dont la structure cationique est de formule:
[(CH(CH₃)₂-Φ-)-I -(-R¹)]⁺ (I)
dans laquelle le symbole R¹ représente le radical -Φ- R², R² étant un radical alkyle linéaire ou ramifié comprenant de 1 à 20 atomes de carbone, de préférence 1 à 15 atomes de carbone.

2. Procédé de préparation selon la revendication 1 **caractérisé en ce que** la structure cationique du sel d'onium est de formule [(CH(CH₃)₂-Φ-)-I-Φ-CH₃]⁺.

3. Procédé de préparation selon la revendication 1 ou 2 **caractérisé en ce que** l'amorceur est un sel d'onium dans lequel la structure anionique est choisie parmi le groupe constitué de Cl⁻, Br⁻, BF₄⁻, PF₆⁻, CF₃SO₃⁻, N(SO₂CF₃)₂^{-,} C(SO2CF₃)₂⁻, B(C₆F₅)₄^{-,}B(PhOCF₃)₄⁻, SbF₆⁻ et/ou AsF₆⁻.

4. Procédé de préparation selon l'une quelconque des revendications précédentes **caractérisé en ce que** l'amorceur est un sel d'onium choisi dans le groupe constitué de
- [CH₃-Φ-I-Φ-CH(CH₃)₂]⁺ Cl⁻,
- [CH₃-Φ-I-Φ-CH(CH₃)₂]⁺ PF₆⁻,
- [CH₃-Φ-I-Φ-CH(CH₃)₂]^{+,}B(C₆F₅)₄⁻,
- [CH₃-Φ-I-Φ-CH(CH₃)₂]⁺B(PhOCF₃)₄⁻,

5. Procédé de préparation selon l'une quelconque des revendications précédentes **caractérisé en ce que** la réticulation est effectuée sous rayonnement ultraviolet et/ou sous rayonnement par faisceau d'électrons.

6. Procédé de préparation selon l'une quelconque des revendications précédentes **caractérisé en ce que** les monomères, oligomères et polymères à groupements organofonctionnels sont des polyorganosiloxanes constitués de motifs de formule (II) et terminés par des motifs de formule (III) ou cycliques constitués de motifs de formule (II) représentées ci-dessous : 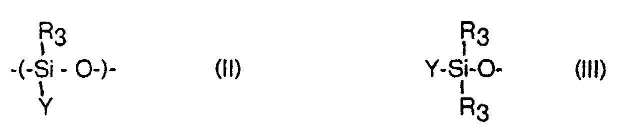 dans lesquelles :
- les symboles R₃ sont semblables ou différents et représentent :
· un radical alkyle linéaire ou ramifié contenant 1 à 8 atomes de carbone, éventuellement substitué par au moins un halogène, de préférence le fluor, les radicaux alkyle étant de préférence méthyle, éthyle, propyle, octyle et 3,3,3-trifluoropropyle,
· un radical cycloalkyle contenant entre 5 et 8 atomes de carbone cycliques, éventuellement substitué,
· un radical aryle contenant entre 6 et 12 atomes de carbone pouvant être substitué, de préférence phényle ou dichlorophényle,
· une partie aralkyle ayant une partie alkyle contenant entre 5 et 14 atomes de carbone et une partie aryle contenant entre 6 et 12 atomes de carbone, substituée éventuellement sur la partie aryle par des halogènes, des alkyles et/ou des alkoxyles contenant 1 à 3 atomes de carbone,
- les symboles Y sont semblables ou différents et représentent :
· le groupement R₃, un radical hydrogène et/ou
· un groupement organofonctionnel réticulable par voie cationique, de préférence un groupement époxyfonctionnel et/ou vinyloxyfonctionnel, relié au silicium du polyorganosiloxane par l'intermédiaire d'un radical divalent contenant de 2 à 20 atomes de carbone et pouvant contenir au moins un hétéroatome, de préférence de l'oxygène,
· et l'un au moins des symboles Y représentant un groupement organique fonctionnel réticulable par voie cationique.

7. Procédé de préparation selon la revendication 6 **caractérisé en ce que** au moins un des symboles R₃ des polyorganosiloxanes utilisés représente un radical phényle, tolyle ou dichlorophényle.

8. Procédé de préparation selon l'une quelconque des revendications 1 à 5 **caractérisé en ce que** les compositions réticulables comprennent des monomères, oligomères et/ou polymères à groupements organofonctionnels choisis parmi ceux ayant des fonctions réactives époxy, acrylate, alcényl-éther et/ou alcool.

9. Procédé de préparation selon la revendication 8 **caractérisé en ce que** les monomères, oligomères et/ou polymères à groupements organofonctionnels appartiennent à au moins l'une des espèces suivantes :
α_{1.1} époxydes cycloaliphatiques, pris à eux seuls ou en mélange entre eux :
- les époxydes du type 3,4-époxycyclohexylméthyl-3',4'-époxycyclohexane carboxylate : 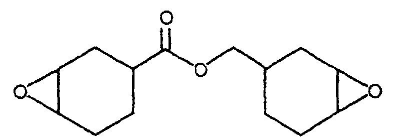
- ou Bis(3,4époxycyclohexyl)adipate, étant particulièrement préférés ;
α_{1.2} époxydes non cycloaliphatiques, pris à eux seuls ou en mélange entre eux
• les époxydes du type de ceux résultant de la condensation de Bisphénol A et d'épichlorhydrine et du type :
- di et triglycidyléthers de Bisphénol A alcoxylé de 1,6 hexane diol, de glycérol, de néopentylglycol et de propane triméthylol,
- ou diglycidyléthers de Bisphénol A,
• les époxydes d'alpha-oléfines, époxyde NOVOLAC, huile de soja et de lin, polybutadiène époxydé,
étant particulièrement préférés ;
α₂ acrylates, pris à eux seuls ou en mélange entre eux ; e.g. :
• acrylates époxydés, de préférence l'oligomère de Bisphénol-A-époxydiacrylate,
• acrylo-glycéro-polyester, de préférence mélange d'oligomère d'acrylate trifonctionnel obtenu à partir de glycérol et de polyester,
• acrylates multifonctionnels, de préférence pentaérythritol triacrylate (PETA), triméthytol propane triacrylate (TMPTA), 1,6-hexanediol diacrylate (HDODA), triméthylolpropane éthoxylate triacrylate, thiodiéthylèneglycol diacrylate, tétra éthylène glycol diacrylate (TTEGDA), tripropylène-glycol diacrylate (TRPGDA), triéthylèneglycol diacrylate (TREGDA), triméthylpropane triméthacrylate (TMPTMA),
• acrylo-uréthanes,
• acrylo-polyéthers,
• acrylo-polyesters,
• polyesters insaturés,
• acrylo-acryliques,
étant particulièrement préférés ;
α₃ alcényl-éthers, linéaires ou cycliques, pris à eux seuls ou en mélange entre eux:
• les vinyls-éthers, en particulier l'éther de triéthylène glycol divinylique (RAPIDCURE® CHVE-3, GAF Chemicals Corp.), les éthers vinyliques cycliques ou les tétramères et/ou dimères d'acroléines, et le vinyl-ether de formule suivante: 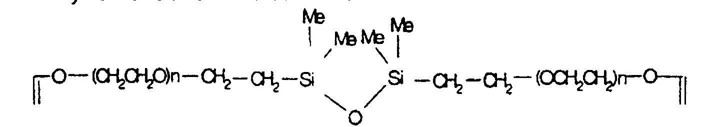
• les propényls-éthers,
• et les butényls-éthers étant, plus spécialement préférés,
α₄ les polyols : pris à eux seuls ou en mélange entre eux, et de préférence le composé de formule ci dessous:

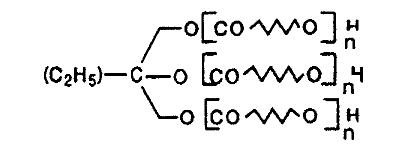

10. Procédé de préparation selon l'une quelconque des revendications 8 ou 9 **caractérisé en ce que** les compositions réticulables comprennent en outre des monomères, oligomères et/ou polymères à groupements organofonctionnels définis en accord avec la revendication 6.

11. Résine susceptible d'être obtenue à partir du procédé de préparation selon l'une quelconque des revendications 1 à 10.

12. Revêtement anti-adhérant non toxique à base de résine selon la revendication 11.

13. Encre non toxique à base de résine selon la revendication 11.

14. Article dont une surface au moins est revêtue de résine de nature organique et/ou de nature silicone selon la revendication 11.

15. Utilisation de résine selon la revendication 11 pour la fabrication de revêtement ou d'encre non toxiques.

16. Utilisation selon la revendication 15 **caractérisée en ce que** le revêtement et/ou l'encre sont à application alimentaire.

17. Utilisation de sel d'onium en tant que système amorceur non toxique pour la préparation de composition réticulable **caractérisée en ce que** la structure cationique du sel d'onium est de formule :
[(CH(CH₃)₂-Φ-)-I-(-R¹)]⁺ (I)
dans laquelle le symbole R¹ représente le radical -Φ- R², R² étant un radical alkyle linéaire ou ramifié comprenant de 1 à 20 atomes de carbone, de préférence 1 à 15 atomes de carbone.

18. Utilisation du sel d'onium selon la revendication 17 **caractérisée en ce que** la structure cationique du sel d'onium est de formule:
[(CH(CH₃)₂-Φ-)-I-Φ-CH₃]⁺.

19. Utilisation selon la revendication 17 ou 18 **caractérisée en ce que** la composition réticulable est préparée en vue de fabriquer des revêtements anti-adhérants non toxiques.

20. Utilisation selon la revendication 18 **caractérisée en ce que** les revêtements anti-adhérants non toxiques sont à application alimentaire.

## Claims

1. Process for preparing non-toxic resins from compositions comprising monomers, oligomers and/or polymers containing organofunctional groups, which can be crosslinked by supplying energy, at least some, preferably all, of which is supplied by UV radiation with a wavelength of between 200 and 400 nanometres, in the presence of an initiator system, **characterized in that** the initiator system is an onium salt whose cationic structure is of the formula:
[(CH(CH₃)₂-Φ-)- I -(-R¹)]⁺ (I)
in which the symbol R¹ represents a radical -φ-R², R² being a linear or branched alkyl radical comprising from 1 to 20 carbon atoms, preferably 1 to 15 carbon atoms.

2. Preparation process according to Claim 1, **characterized in that** the cationic structure of the onium salt is of the formula
[(CH(CH₃)₂-Φ-)- I -Φ- CH₃]⁺.

3. Preparation process according to Claim 1 or 2, **characterized in that** the initiator is an onium salt in which the anionic structure is chosen from the group consisting of Cl⁻, Br⁻, BF₄⁻, PF₆⁻, CF₃SO₃⁻, N(SO₂CF₃)₂^{-,} C(SO₂CF₃)₂⁻, B(C₆F₅)₄^{-,} B(PhOCF₃)₄⁻, SbF₆⁻ and/or AsF₆⁻.

4. Preparation process according to any one of the preceding claims, **characterized in that** the initiator is an onium salt chosen from the group consisting of
- [CH₃-Φ-I-Φ- CH(CH₃)₂]⁺ Cl⁻,
- [CH₃-Φ-I-Φ- CH(CH₃)₂]⁺ PF₆⁻,
- [CH₃-Φ-I-Φ- CH(CH₃)₂]⁺, B(C₆F₅)₄^{-,}
- [CH₃-Φ-I-Φ- CH(CH₃)₂]⁺ B(PhOCF₃)₄⁻,

5. Preparation process according to any one of the preceding claims, **characterized in that** the crosslinking is carried out under ultraviolet irradiation and/or under irradiation with an electron beam.

6. Preparation process according to any one of the preceding claims, **characterized in that** the monomers, oligomers and polymers containing organofunctional groups are polyorganosiloxanes consisting of units of formula (II) ending with units of formula (III), or are cyclic and consist of units of formula (II) represented below: 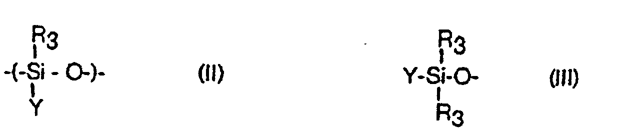 in which
- the symbols R₃ are identical or different and represent:
· a linear or branched alkyl radical containing 1 to 8 carbon atoms, optionally substituted with at least one halogen, preferably fluorine, the alkyl radicals preferably being methyl, ethyl, propyl, octyl and 3,3,3-trifluoropropyl,
· a cycloalkyl radical containing between 5 and 8 carbon atoms, as a ring which is optionally substituted,
· an aryl radical containing between 6 and 12 carbon atoms which may be substituted, preferably phenyl or dichlorophenyl,
· an aralkyl portion with an alkyl portion containing between 5 and 14 carbon atoms and an aryl portion containing between 6 and 12 carbon atoms, optionally substituted on the aryl portion with halogens, alkyls and/or alkoxys containing 1 to 3 carbon atoms,
- the symbols Y are identical or different and represent:
· the group R₃, a hydrogen radical and/or
· an organofunctional group which can undergo cationic crosslinking, preferably an epoxyfunctional and/or vinyloxyfunctional group linked to the silicon of the polyorganosiloxane via a divalent radical containing from 2 to 20 carbon atoms and which can contain at least one hetero atom, preferably oxygen, and
· at least one of the symbols Y representing an organofunctional group which can undergo cationic crosslinking.

7. Preparation process according to Claim 6, **characterized in that** at least one of the symbols R₃ of the polyorganosiloxanes used represents a phenyl, tolyl or dichlorophenyl radical.

8. Preparation process according to any one of Claims 1 to 5, **characterized in that** the crosslinkable compositions comprise monomers, oligomers and/or polymers containing organofunctional groups chosen from those containing epoxy, acrylate, alkenyl ether and/or alcohol reactive functions.

9. Preparation process according to Claim 8, **characterized in that** the monomers, oligomers and/or polymers containing organofunctional groups belong to at least one of the following species:
α1.1 cycloaliphatic epoxides, taken alone or as a mixture with each other:
- epoxides of the 3,4-epoxycyclohexylmethyl 3',4'-epoxycyclohexanecarboxylate type: 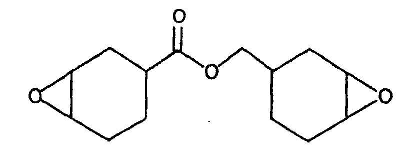
- or bis(3,4-epoxycyclohexyl) adipate, being particularly preferred;
α1.2 non-cycloaliphatic epoxides, taken alone or as a mixture with each other:
· epoxides of the type resulting from the coupling of bisphenol A and epichlorohydrin and of the type:
- di- and triglycidyl ethers of alkoxylated bisphenol A of 1,6-hexanediol, of glycerol, of neopentyl glycol and of trimethylolpropane,
- or bisphenol A diglycidyl ethers,
· α-olefin epoxides, Novolac epoxide, epoxidized soybean oil, epoxidized flax oil and epoxidized polybutadiene,
being particularly preferred;
α2 acrylates, taken alone or as a mixture with each other, e.g.:
· epoxidized acrylates, preferably the oligomer of bisphenol-A-epoxydiacrylate,
· acrylo-glycero-polyester, preferably trifunctional acrylate oligomer mixture obtained from glycerol and polyester,
· multifunctional acrylates, preferably pentaerythrityl triacrylate (PETA), trimethylolpropane triacrylate (TMPTA), 1,6-hexanediol diacrylate (HDODA), trimethylolpropane ethoxylate triacrylate, thiodiethylene glycol diacrylate, tetraethylene glycol diacrylate (TTEGDA), tripropylene glycol diacrylate (TRPGDA), triethylene glycol diacrylate (TREGDA) or trimethylpropane trimethacrylate (TMPTMA),
· acrylo-urethanes,
· acrylo-polyethers,
· acrylo-polyesters,
· unsaturated polyesters,
· acrylo-acrylics,
being particularly preferred;
α3 linear or cyclic alkenyl ethers, taken alone or as a mixture with each other: vinyl ethers, in particular triethylene glycol divinyl ether (Rapidcure® CHVE-3, GAF Chemicals Corp.), cyclic vinyl ethers or acrolein tetramers and/or dimers, and the vinyl ether of the following formula: 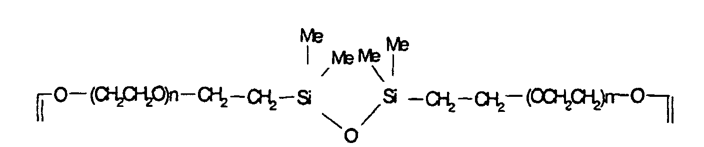
· propenyl ethers,
· and butenyl ethers being more especially preferred,
α4 polyols, taken alone or as a mixture with each other, and preferably the compound of the formula below:

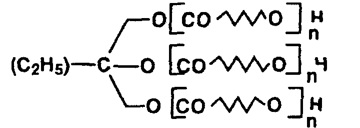

10. Preparation process according to either of Claims 8 and 9, **characterized in that** the crosslinkable compositions also comprise monomers, oligomers and/or polymers containing organofunctional groups defined in accordance with Claim 6.

11. Resin which can be obtained from the preparation process according to any one of Claims 1 to 10.

12. Non-toxic, non-stick coating based on resin according to Claim 11.

13. Non-toxic ink based on resin according to Claim 11.

14. Article, at least one surface of which is coated with resin of organic nature and/or silicone nature according to Claim 11.

15. Use of resin according to Claim 11 for the manufacture of non-toxic ink or coating.

16. Use according to Claim 15, **characterized in that** the coating and/or ink are for food use.

17. Use of onium salt as a non-toxic initiator system for the preparation of a crosslinkable composition, **characterized in that** the cationic structure of the onium salt is of the formula:
[(CH(CH₃)₂-Φ-)- I -(-R¹)]⁺ (I)
in which the symbol R¹ represents a radical -φ-R², R² being a linear or branched alkyl radical comprising from 1 to 20 carbon atoms, preferably 1 to 15 carbon atoms.

18. Use of the onium salt according to Claim 17, **characterized in that** the cationic structure of the onium salt is of the formula:
[(CH(CH₃)₂-Φ-)- I -Φ-CH₃]⁺.

19. Use according to Claim 17 or 18, **characterized in that** the crosslinkable composition is prepared for the purpose of manufacturing non-stick, non-toxic coatings.

20. Use according to Claim 18, **characterized in that** the non-toxic, non-stick coatings are for food use.

## Patentansprüche

1. Verfahren zur Herstellung von nicht toxischen Harzen aus Zusammensetzungen, die Monomere, Oligomere und/oder Polymere mit organofunktionellen Gruppen umfassen, die durch Energiezufuhr, von der wenigstens ein Teil, vorzugsweise die Gesamtheit, durch eine UV-Strahlung mit einer Wellenlänge zwischen 200 und 400 Nanometer geliefert wird, in Gegenwart eines Initiatorsystems vernetzbar sind, **dadurch gekennzeichnet, dass** das Initiatorsystem ein Oniumsalz ist, dessen Kationenstruktur die Formel:
[(CH(CH₃)₂-Φ-)-I-(-R¹)]⁺ (I)
besitzt, worin das Symbol R¹ den Rest **-Φ**-R² darstellt, wobei R² ein linearer oder verzweigter Alkylrest mit 1 bis 20 Kohlenstoffatomen, vorzugsweise 1 bis 15 Kohlenstoffatomen ist

2. Herstellungsverfahren gemäß Anspruch 1, **dadurch gekennzeichnet, dass** die Kationenstruktur des Oniumsalzes die Formel [(CH(CH₃)₂-Φ-)-I-Φ-CH₃]⁺ besitzt.

3. Herstellungsverfahren gemäß Anspruch 1 oder 2, **dadurch gekennzeichnet dass** der Initiator ein Oniumsatz ist, worin die Anionenstruktur aus der Gruppe ausgewählt ist, die aus Cl⁻, Br⁻, BF₄⁻, PF₆⁻ , CF₃SO₃⁻, N(SO₂CF₃)₂⁻, C(SO₂CF₃)₂⁻, B(C₆F₅)₄⁻, B(PhOCF₃)₄⁻, SbF₆⁻, und/oder AsF₆⁻ besteht.

4. Hersteilungsverfahren gemäß einem der vorhergehenden Ansprüche, **dadurch gekennzeichnet, dass** der Initiator ein Oniumsalz ist, das ausgewählt ist aus der Gruppe, die besteht aus
-[CH₃-Φ-I-Φ-CH(CH₃)₂]⁺ CI⁻,
-[(CH₃-Φ-I-Φ-CH(CH₃)₂]⁺ PF₆⁻,
-[CH₃-Φ-I-Φ-CH(CH₃)₂]⁺ B(C₆F₅)₄⁻.
-[CH₃-Φ-I-Φ-CH(CH₃)₂]⁺ B(PhOCF₃)₄-.

5. Herstellungsverfahren gemäß einem der vorhergehenden Ansprüche, **dadurch gekennzeichnet, dass** die Vernetzung unter Ultraviolettstrahlung und/oder unter Bestrahlung mit Elektronenstrahlen ausgeführt wird.

6. Herstellungsverfahren gemäß einem der vorhergehenden Ansprüche, **dadurch gekennzeichnet, dass** die Monomere, Oligomere und Polymere mit organofunktionellen Gruppen Polyorganosiloxane, die aus Einheiten der Formel (II) bestehen und mit Einheiten der Formel (III) enden, oder cyclische Polyorganosiloxane sind, die aus Einheiten der Formel (II) bestehen, die im Folgenden dargestellt sind: 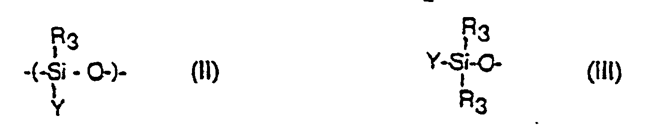 worin:
- die Symbole R₃ gleich oder verschieden sind und darstellen:
• einen linearen oder verzweigten Alkylrest mit 1 bis 8 Kohlenstoffatomen, gegebenenfalls substituiert mit wenigstens einem Halogen, vorzugsweise Fluor, wobei die Alkylreste vorzugsweise Methyl, Ethyl, Propyl, Octyl und 3,3,3-Trifluorpropyl sind,
• einen Cycloalkylrest, der zwischen 5 und 8 cyclische Kohlenstoffatome enthält, gegebenenfalls substituiert,
• einen Arylrest, der zwischen 6 und 12 Kohlenstoffatome enthält, der substituiert sein kann, vorzugsweise Phenyl oder Dichlorphenyl.
• einen Aralkylteil mit einem Alkylteil, der zwischen 5 und 14 Kohlenstoffatome enthält, und einem Arylteil, der zwischen 6 und 12 Kohlenstoffatome enthält, gegebenenfalls am Arylteil mit Halogenen, Alkylen und/oder Alkoxylen mit 1 bis 3 Kohlenstoffatomen substituiert,
- die Symbole Y gleich oder verschieden sind und darstellen:
• die Gruppe R₃, einen Wasserstoffrest und/oder
• eine kationisch vernetzbare organofunktionelle Gruppe, vorzugsweise eine epoxyfunktionelle und/oder vinyloxyfunktionelle Gruppe, die an das Silicium des Polyorganosiloxans über einen zweiwertigen Rest mit 2 bis 20 Kohlenstoffatomen gebunden ist und die wenigstens ein Heteroatom, vorzugsweise Sauerstoff, enthalten kann,
• und wobei wenigstens eins der Symbole Y eine kationisch vernetzbare funktionelle organische Gruppe darstellt

7. Herstellungsverfahren gemäß Anspruch 6, **dadurch gekennzeichnet, dass** wenigstens eins der Symbole R₃ der verwendeten Polyorganosiloxane einen Phenyl-, Tolyl- oder Dichlorphenylrest darstellt

8. Herstellungsverfahren gemäß einem der Ansprüche 1 bis 5, **dadurch gekennzeichnet, dass** die vemetzbaren Zusammensetzungen Monomere, Oligomere und/oder Polymere mit organofunktionellen Gruppen umfassen, die ausgewählt sind unter denjenigen mit reaktiven Epoxy-, Acrylat-, Alkenylether- und/oder Alkoholfunktionen.

9. Herstellungsverfahren gemäß Anspruch 8, **dadurch gekennzeichnet, dass** die Monomere, Oligomere und/oder Polymere mit organofunktionellen Gruppen wenigstens einer der folgenden Spezies angehören:
α_{1.1} cycloaliphatische Epoxide, allein genommen oder im Gemisch miteinander
- die Epoxide vom Typ 3,4-Epoxycyclohexylmethyl-3',4'-epoxycyclohexancarboxylat: 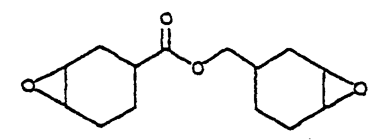
- oder Bis(3,4-epoxycyclohexyl)adipat, die besonders bevorzugt sind;
α_{1.2} nicht cycloaliphatische Epoxide, allein genommen oder im Gemisch miteinander
• die Epoxide vom Typ derjenigen, die sich aus der Kondensation von Bisphenol A und Epichlorhydrin ergeben, und vom Typ:
- Di- und Triglycidylether von mit 1,6-Hexandiol alkoxyliertem Bisphenol A, von Glycerin, von Neopentylglykol und von Trimethylolpropan,
- oder Diglycidylether von Bisphenol A,
• die Epoxide von alpha-Olefinen, NOVOLAC-Epoxid, Soja- und Leinöl, epoxydiertes Polybutadien,
die besonders bevorzugt sind;
α₂ Acrylate, allein genommen oder im Gemisch miteinander; z. B.:
• Epoxidacrylate, vorzugsweise das Oligomer von Bisphenol-A-epoxydiacrylat,
• Acryl-Glycerin-Polyester, vorzugsweise Gemisch von trifunktionellem Acrylatoligomer, das erhalten wird aus Glycerin und Polyester,
• multifunktionelle Acrylate, vorzugsweise Pentaerythrittriacrylat (PETA), Trimethylolpropantriacrylat (TMPTA), 1,6-Hexandioldiacrylat (HDODA), ethoxyliertes Trimethylolpropantriacrylat, Thiodiethylenglykoldiacrylat, Tetraethylenglykoldiacrylat (TTEGDA), Tripropylenglykoldiacrylat (TRPGDA), Triethylenglykoldiacrylat (TREGDA), Trimethylpropantrimethacrylat (TMPTMA),
• Urethanacrylate,
• Polyetheracrylate,
• Polyesteracrylate,
• ungesättigte Polyester,
• acrylierte Acrylate,
die besonders bevorzugt sind;
α₃ lineare oder cyclische Alkenylether, allein genommen oder im Gemisch miteinander:
• die Vinylether, insbesondere Triethylenglykoldivinylether (RAPIDCURE® CHVE-3, GAF Chemicals Corp.), die cyclischen Vinylether oder die Tetramere und/oder Dimere von Acroleinen und der Vinylether der folgenden Formel: 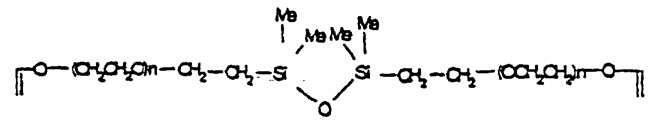
• die Propenylether,
• und die Butenylether, die spezieller empfohlen sind,
α₄ die Polyole, allein genommen oder im Gemisch miteinander und vorzugsweise die Verbindung der folgenden Formel:

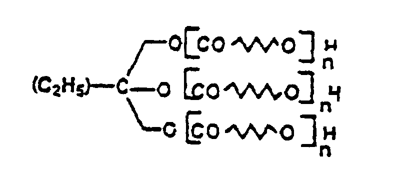

10. Herstellungsverfahren gemäß einem der Ansprüche 8 oder 9, **dadurch gekennzeichnet, dass** die vemetzbaren Zusammensetzungen außerdem Monomere, Oligomere und/oder Polymere mit organofunktionellen Gruppen, die In Übereinstimmung mit Anspruch 6 definiert sind, umfassen.

11. Harz, das aus dem Herstellungsverfahren gemäß einem der Ansprüche 1 bis 10 erhalten werden kann.

12. Nicht toxische Antihaftbeschichtung auf der Basis von Harz gemäß Anspruch 11.

13. Nicht toxische Druckfarbe auf der Basis von Harz gemäß Anspruch 11.

14. Gegenstand, bei dem wenigstens eine Oberfläche mit Harz organischer Art und/oder der Art Silikon gemäß Anspruch 11 beschichtet ist.

15. Verwendung von Harz gemäß Anspruch 11 für die Herstellung von nicht toxischer Beschichtung oder Druckfarbe.

16. Verwendung gemäß Anspruch 15, **dadurch gekennzeichnet, dass** die Beschichtung und/oder die Druckfarbe für die Anwendung im Lebensmittelbereich bestimmt sind.

17. Verwendung von Oniumsalz als nicht toxisches Initiatorsystem für die Herstellung einer vernetzbaren Zusammensetzung, **dadurch gekennzeichnet, dass** die Kationenstruktur des Oniumsalzes die Formel:
[(CH(CH₃)₂-Φ-)-I-(R¹)]⁺ (I)
besitzt, worin das Symbol R¹ den Rest -Φ-R² darstellt, wobei R² ein linearer oder verzweigter Alkylrest mit 1 bis 20 Kohlenstoffatomen, vorzugsweise 1 bis 15 Kohlenstoffatomen ist

18. Verwendung des Oniumsalzes gemäß Anspruch 17, **dadurch gekennzeichnet, dass** die Kationenstruktur des Oniumsalzes die Formel [(CH(CH₃)₂-Φ-)-I-Φ-CH₃]⁺ besitzt.

19. Verwendung gemäß Anspruch 17 oder 18, **dadurch gekennzeichnet, dass** die vernetzbare Zusammensetzung im Hinblick auf die Herstellung von nicht toxischen Antihaftbeschichtungen hergestellt wird.

20. Verwendung gemäß Anspruch 18, **dadurch gekennzeichnet, dass** die nicht toxischen Antihaftbeschichtungen für die Anwendung im Lebensmittelbereich bestimmt sind.
